# EUROPEAN PATENT SPECIFICATION

(11) **EP 2 568 870 B1**
(45) Date of publication and mention of the grant of the patent: **02.05.2018**
(21) Application number: 11762051.8
(22) Date of filing: 30.03.2011
(51) Int. Cl.: G01B 11/25, G01B 11/245, A61B 1/00, A61B 1/05, A61B 5/107, A61B 1/227, A61B 1/06

(54) **SCANNING OF CAVITIES WITH RESTRICTED ACCESSIBILITY**
SCANNEN VON EINGESCHRÄNKT ZUGÄNGLICHEN HOHLRÄUMEN
BALAYAGE DE CAVITÉS À ACCESSIBILITÉ RÉDUITE

(30) Priority: 30.03.2010 US 318882 P; 30.03.2010 DK 201000264
(43) Date of publication of application: 20.03.2013
(73) Proprietor: 3Shape A/S, 1060 Copenhagen K (DK)
(72) Inventor: VAN DER POEL, Mike, 2610 Rødovre (DK); HOLLENBECK, Karl-Josef, 2100 Copenhagen Ø (DK); FISCHER, David, 3660 Stenløse (DK); VINTHER, Michael, 2300 Copenhagen S (DK)
(74) Representative: Guardian IP Consulting I/S
(86) International application number: PCT/DK2011/050103
(87) International publication number: WO 2011/120526

(56) References cited:
- WO-A1-02/16867
- WO-A1-02/16867
- WO-A1-02/071794
- WO-A1-02/071794
- WO-A1-2010/145669
- WO-A2-02/16865
- WO-A2-02/16865
- WANG ET AL: "Filling holes on locally smooth surfaces reconstructed from point clouds", IMAGE AND VISION COMPUTING, ELSEVIER, GUILDFORD, GB, vol. 25, no. 1, 2 November 2006 (2006-11-02), pages 103-113, XP005846922, ISSN: 0262-8856, DOI: 10.1016/J.IMAVIS.2005.12.006

## Description

The invention relates to the creation of high precision three-dimensional replicas of real objects. The invention specifically concerns the three-dimensional scanning of interior surfaces or cavities of limited dimensions or with restricted accessibility. Furthermore, the invention relates in particular to scanning the human ear and ear canal.

Systems for three-dimensional optical scanning are well known in the prior art. They typically comprise one or more light sources projecting a structured light pattern on the object to be scanned, one or more cameras and data processing equipment to convert the recorded image coordinates to three dimensional coordinates using state of the art software. Usually, only a part of the object is scanned in a single scan. To create a full scan the object, camera and light source need to be move relative to each other.

Accuracy and precision are of utmost importance in many applications, e.g. when the scan data is used to model an object that must fit precisely into another part. Such applications are e.g. devices for the ear canal such as hearing aids, dental implants and other prostheses for the body. For hearing aid shells, sub-millimeter precision is required lest the shell cause irritation, acoustic feedback and possibly infection to the epidermis of the ear canal. For dental implants the precision requirement is even greater, since a human being can detect differences less than 1/10 of a millimeter when biting. Therefore systematic or random errors in the calibration and/or performance of scanners for these uses can be serious. This has hitherto limited the use of scanning in the modeling of such implants and shells.

WO200216865 describes transformation of image co-ordinates into 3D real world co-ordinates where sufficiently accurate results are obtained without handling extremely large data sets. Instead a reduced number of observations are provided and interpolation provides a set of 3D real world co-ordinates with an increased spatial resolution compared to the original data.

WO2002071794 describes modification of a digital 3D model of an ear impression. An existing surface of the digital 3D model is deleted and a new surface is constructed by a lofting procedure.
Wang et al. describes a method for creating 3D models of real scenes when not all parts of the scene are sampled due to surface reflectance properties, occlusions or accessibility limitations. In a first aspect the present invention provides a method for scanning interior surfaces, where the method comprises:
- providing a probe shaped scanner having an axis, where the probe shaped scanner comprises:
   - at least one light source configured to create and project structured light, and
   - at least one camera configured to record 2D images;
- entering said probe shaped scanner into a cavity of an object, where said cavity is bounded by an interior surface of the object;
- creating and projecting structured light from said light source of the probe producing a pattern on the interior surface of the object;
- recording a series of 2D images of the reflection of the pattern from the interior surface using said camera;
- combining said series of 2D images to obtain 3D real world coordinates of the interior surface; and
- registering one or more holes in the 3D real world coordinates corresponding to areas of the interior surface where visual access is blocked due to the geometrical form of the interior surface; and
- providing data and processing said data to create 3D real world coordinates for the areas of the interior surface where said visual access is blocked.

The method is suitable for scanning partly obstructed interior surfaces, where visual access to part of the interior surface is blocked due to the geometrical form of the interior surface where e.g. bends may prevent visual access to a part of the interior surface.

In some embodiments, the method comprises:
- providing that the camera comprises a plurality of sensor elements or a plurality of groups of sensor elements;
- varying the focus plane of the pattern over a range of focus plane positions while maintaining a substantially fixed spatial relation of the camera and said interior surface,
where the combining of 2D images comprises determining by analysis of said 2D images the in-focus position(s) of the focus plane for:
a) each of a plurality of sensor elements in the camera for said range of focus plane positions, or
b) each of a plurality of groups of the sensor elements in the camera for said range of focus planes,
and where said 3D real world coordinates are from said in-focus positions. In a second aspect the present invention provides a scanner system for three-dimensional scanning of interior surfaces, said scanner system comprising:
- a probe shape scanner configured to be entered into a cavity, said probe shaped scanner comprising
   - at least one light source adapted to create and project structured light producing a pattern on the interior surface of an object, where said light source emits light from a point of emission; and
   - at least one camera, adapted to record 2D images of the pattern, where the camera accumulates light at a point of accumulation;
- a data conversion device adapted to convert 2D images into 3D real world coordinates,
- a data processing device configured to register one or more holes in the 3D real world coordinates corresponding to areas of the interior surface where visual access is blocked due to the geometrical form of the interior surface, and to provide data and process said data to create 3D real world coordinates for the areas of the interior surface where said visual access is blocked. Disclosed is furthermore a method (not claimed) for scanning interior surfaces comprising the
- entering a probe shaped scanner having an axis into a cavity,
- creating and projecting structured light from a light source on the probe producing a pattern on an interior surface of an object, and at a camera of the probe, recording 2D images of the pattern reflected from the interior surface, thereby performing a scan around the axis of the probe,
- determining 2D coordinates of the images of the pattern,
- combining a series of images to obtain 3D real world coordinates of the interior surface, and
- processing data such that surface information from areas of the surface, where image scanning is not complete, is created.

Consequently, it is an advantage that surface information can be obtained for e.g. areas where there are no 2D images recorded, due to e.g. holes of surface information, by e.g. using kinds of data other than image data.
In some embodiments, the data that are provided and processed to create surface information, are obtained from the recorded 2D images.
In some embodiments, the probe shaped scanner has an axis and where a scan around the axis is performed.

In the context of the present invention, the phrase "hole in the surface information" relates to a part of the surface where data is lacking, and such a lack of data occurs where visual access to the interior surface is blocked due to the geometrical form of the interior surface. Furthermore it is an advantage that surface information can be obtained even if the surface in e.g. the ear is not clean, such that 2D images do not correctly show the recorded surface, e.g., if there are foreign objects, such as ear wax in the ear covering parts of the surface, or if there are other foreign objects such as hair, scars or animals in the ear diminishing the quality of the scanning. Thus foreign object may be hair, ear wax, small animals such as insects, eggs from insects, pearls, sand, dirt, pimples, scars etc.
Consequently, it may be an advantage that prior to scanning, the patient's ear need not be rinsed to remove foreign objects such as cerumen or ear wax. Hereby the patient does not need to visit a doctor to have his/her ear rinsed. In some cases it may be advantageous to apply a diffusively reflecting material to the surface of the ear canal if the appearance is too glossy.
Both the ear canal and the external part of the ear, called the pinna, may be scanned.

It is an advantage that one scan can be performed in less than 1 minute.

The axis of the scanner may be the longitudinal axis of the scanner and/or the optical axis of the scanner. The optical axis of the scanner may be defined by the optical axis of the camera or the optical axis of the light source

It is an advantage to scan the ear and the ear canal directly with a scanner, instead of providing an impression of the ear canal, and then scanning the impression. The silicone used when making an impression expands the ear and may thus not give a correct impression.

It is a further advantage with regard to ear scanning that the improved fit of the hearing aid shells according to the present invention compared to prior art hearing aid shells means that the frequent problem of acoustic feedback in hearing aids is minimized.

Furthermore it is an advantage that the direct scanning of the ear significantly reduces the production cost of hearing aids, since the impressions used today are rendered superfluous. Obviating the impression removes the handling and mailing cost and cuts down the production time and improves flexibility.

Thus it is an advantage that the method allows for easy scanning of interior surfaces of objects which cannot be scanned with high precision using prior art scanning methods.

Preferably the method is carried out with a scanner according to the invention.

According to one aspect the invention relates to a method for 3D modeling and production comprising obtaining 3D real world coordinates of an interior surface of a cavity provided using the method according to the invention, and creating a piece adapted to fit into the cavity.
Thereby, the steps for manufacturing the piece are reduced to the absolute minimum and an essentially perfect fit of the piece can be obtained. The interior surface may be scanned a number of times, such as under different conditions affecting the geometry of the interior surface. Thereby the variations in the dimensions of the interior surface can be recorded. This is very cumbersome using the prior art techniques.

Once the data are recorded the piece may be manufactured using any automatic manufacturing technique such as milling. More preferably the modeling technique comprises 3Dimensional printing, stereo lithography, selective laser sintering, laminated object modeling, inkjet modeling, fused deposition modeling, or nano-printing. A common feature of these techniques is that only the required amount of material is used and that it is easier to produce complex models such as devices for the ear and/or ear canal and/or dental implants.

The devices for the ear may comprise a hearing aid, a mobile phone, a loud speaker, noise protection, a microphone, communication devices, a tinnitus masker or a tinnitus masking device such as the ones described in US 5,325,872 and WO 91/17638.

In some embodiments holes in the surface information are registered.
When holes in the surface information of the physical object are registered, other ways of determining the surface or the contour of the surface at these areas can be applied such that all areas will be covered, imaged, detected, or interpolated in one way or the other for acquiring surface information.

In some embodiments holes in the surface information are closed by fitting to higher-order mathematical surfaces, such as second-order, third-order, fourth-order etc.
The information from neighbour areas etc can be used to infer the contour at holes. Thus the artificial hole closing may be performed by fitting parametric surface such as spline surface or higher-order mathematical surfaces.
In some embodiments holes in the surface information are closed by using information about where there exists no surface.
If complete image data are missing from a region of the ear canal, then knowledge about the ear anatomy indicating that there exists no surface in said particular part of the region can be used to determine where the surface then should be arranged or interpolated. Thus the surface should not be in an area where it is positively detected that there is no surface. Thus it is an advantage to exclude a volume from scans or 2D images where it is certain that there exists no surface.
In some embodiments holes in the surface information are closed by combing image data with data other than image data.
It is an advantage to combine image data with other types of data, e.g. data from other sources or sensors than the image sensor or camera, such as data from a touch sensor, such that the image data can be verified, assisted or supported by the other data.
In some embodiments holes in the surface information are closed by using other data than image data.
It is an advantage to use data from other sources or sensors than the camera providing the image data, if there exist no image data covering an area of the surface.

In some embodiments the surface model obtained from the scan is processed, for example smoothed. Processing can remove noise and outliers in the raw scan data.

In some embodiments other data than image data comprises color, interference, angle of reflected light, and/or data from one or more other sensors than the camera.

In some embodiments the one or more other sensors comprise touch sensor, contact sensor, sonic sensor, and/or temperature sensor.

In some embodiments a part of the data of surface information of the left ear is used as a part of the data of surface information for the right ear, if there are parts of the right ear where surface information has not been acquired, and vice versa.
It is an advantage to use corresponding scan data from one ear to the other ear, because of symmetry between a person's two ears.

A part of the data of surface information of the left ear may be used for verifying data obtained from one the right ear even though no data are missing for that ear, and vice versa.

In some embodiments holes or missing areas in a scan are inferred or interpolated based on previous scans of the person's ear. It is an advantage because especially children with hearing loss require refitted hearing aids frequently as they grow, but the shape of their ear canal may not change as rapidly as its size.

In some embodiments foreign objects in the ear canal are identified.

In some embodiments identifying foreign objects comprises detecting reflections from the foreign object.
It is an advantage because foreign objects such as hair may reflect light very well, and thus the reflections from hair and other foreign objects may be distinct from reflections from skin in the ear.

In some embodiments a foreign object is identified by analyzing the difference in the reflected light from the skin and the foreign object, respectively.
The difference in the reflected light may be the difference in contrast of the reflected light.

In some embodiments a foreign object is identified if it has an overall higher or lower reflectivity than the skin.

In some embodiments the foreign object is identified if there is a sudden change in reflectivity at the boundary between the foreign object and the skin.

In some embodiments the foreign object is identified by means of a combination of its overall higher or lower reflectivity than the skin and the sudden change in reflectivity at the boundary between the foreign object and the skin.

It is an advantage to detect the foreign object by its different reflectivity, because it allows monochromatic or near-monochromatic light to be used. Thus detection of the contrast is better according to the present method than described in prior art US7529577B, which discloses "analyzing the output with respect to spectral composition of the light received at the light sensitive element and identifying foreign object based on the spectral composition of the received light", see claim 1. According to the present method a monochromatic or near-monochromatic light may be used.

In some embodiments the method comprises using monochromatic light to illuminate the surface for analyzing reflected light.
The light or illumination may alternatively be semi-monochromatic or light having more wavelengths.

In some embodiments the method comprises identifying foreign objects by correlating surface data with the 3D position of the specific data.
It is an advantage because the places in the ear where hair and ear wax are typically present may be known in advance. Likewise the places in the ear where hair or ear wax seldom or never are present may also be known in advance. Thus by correlating the predefined knowledge about typical places for hair and ear wax, respectively, with the actual scan, it is possible to either rule out that a foreign object can be hair or ear wax, or possible to determine with a high probability that a detected foreign object is hair or ear wax.

In some embodiments detecting hair comprises having predefined information in the system about where hair normally is arranged in the ear.
It is an advantage because if inconclusive data is obtained from a place in the ear, and this place typically contains hair, then the inconclusive data is likely to stem from hair.

In some embodiments foreign objects in the ear are filtered out from the images of the ear.
It is an advantage that the ear shall not be cleansed and rinsed before scanning the ear, because foreign object such as ear wax is filtered out from the images.

In some embodiments foreign objects are presented to the operator on a display.

In some embodiments the operator can assist in detecting foreign objects by reacting to the data presented on the display. This may be by pointing toward foreign objects or by pointing toward borders between the foreign objects and the skin.

In some embodiments the foreign objects are colored in a different color on the display than the rest of the surface on the image.

In some embodiments detecting hair comprises detecting and deleting single points on the images.
It is an advantage because hair may appear as single points on the images, because a hair is normally not thick enough to provide a real image of it.

In some embodiments detecting hair comprises detecting the root of the hair in the skin.
It is an advantage to detect the root of a hair, because it ends in an enlargement, the hair bulb, which is whiter in color and softer in texture than the shaft, and is lodged in a follicular involution called the hair follicle in the epidermis layer of the skin, and therefore it may be easier to detect the root of a hair than the hair itself, because the skin is different at the point where a hair is growing out.

In some embodiments detecting hair comprises detecting the thickness of the hair.
It is an advantage because hereby it can be determined whether a hair is so thick that it may influence the measurements or whether the hair is thin enough to be disregarded.

In some embodiments detecting hair comprises detecting the movement of hair when the probe touches the hair.

In some embodiments the method comprises determining whether scar tissue is fresh or old, when detecting a foreign object in the form of scar tissue,
It is an advantage because if the scar is fresh, the scar should heal before the ear scanned and a hearing device is applied in the ear.
On the other hand if the scar is old, then a hearing device can be fitted by avoiding that the hearing device applies pressure on the scar, which will be uncomfortable for the user.

The light source may be configured to create and project structured light such that a pattern is produced on a surface exposed to light from the light source.

In some embodiments color filters are applied to the light source.
It is an advantage to use color filters such as red, green and blue, since hereby different colors to be emitted to the surface can be produced using only a single light source.

In some embodiments the light source is adapted to emit different colors.
It is an advantage since the different colors can be used to detect different objects or features of the surface. The wavelengths of the different colors may be close to each other to obtain a good result.

In some embodiments the light source emits multi-colored light.
It is an advantage to use multi-colored light in combination with the patterned light, e.g. chessboard pattern, because each period or region in the pattern can be analysed for each different color.

Furthermore, it is an advantage to use multi-colored light in combination with a pattern with differently colored periods or regions because each period or region in the pattern can be analyzed for each different color without analyzing the spectral composition of the light reflected from the surface.

In some embodiments the light source emits monochromatic or near-monochromatic light but switches between different colors in time. It is an advantage to use light varying in color over a period of time because the pattern can be analyzed for each different color without analyzing the spectral composition of the light reflected from the surface.

In some embodiments the size of regions of the emitted light pattern is 20x20 mm.

In some embodiments the size of the regions of the emitted light pattern is 10x10 mm.

It is an advantage to use a pattern which is two times smaller, since hereby there are four squares within one area instead of one square within the same area, and hereby a better resolution can be achieved.

In some embodiments light with different polarisation is used, such that light with different polarisations is projected onto the interior surface of the object.

In some embodiments the polarization state of the emitted and/or the detected light is controlled.
It is an advantage to control the polarization because hereby skin and in particular hair, which has a high degree of specular reflection, can be distinguished from surfaces with a low degree of specular reflection. Surfaces with a high degree of specular reflection reflect light with a specific relationship between the polarization of the incident and reflected light. In this way surfaces with high degree of specular reflection may be distinguished from surfaces with low degree of specular reflection.

The polarization of reflected light may be controlled by means of e.g. a linear polarization filter or quarter wave plate or filter, which can be added and/or removed from the light path and/or rotated in the light path.

In some embodiments the light source is a laser and light from said laser is guided to impinge on the surface in a small spot.

In some embodiments there is more than one light source in the probe shaped scanner and the different light sources are alternately turned on and turned off.

In some embodiments the light source emits infrared light.

It is an advantage because it may be possible to see right through hair using infrared light.

In some embodiments the optical resolution of the scanner is 100 µm.
It is an advantage to have such a high resolution since hereby as many details of the surface as possible can be resolved. Alternatively, the resolution of the scanner may be 200 µm.

In some embodiments the method comprises detecting the hardness under the skin in different parts of the ear canal.
It is an advantage because a soft part of the ear canal is likely to be tissue, and a hard part of the ear canal is likely to be bone, and if the hearing device has a very tight fit in the ear canal where there is bone, it may be very unpleasant for the user. On the other hand, the hearing device can have a tight fit where there is soft tissue under the skin in the ear canal. So by detecting where there is soft tissue and hard bone in the ear canal, the hearing device can be made to fit very comfortably in the user's ear.

In some embodiments light with different wavelengths is used to detect the hardness under the skin in the ear canal.
It is an advantage because the different layers of skin, bone, tissue etc has different properties regarding absorption and/or scattering for different light wavelengths.

In some embodiments the light used for scanning comprises one or more narrow laser beams co-propagating with a broader light beam. The co-propagating laser beams may be light that is reflected and/or refracted from a prism or a beam-combining element. It is an advantage because the narrow laser beams may be of a different color than the broader light beam. The light from the narrow laser beams reflected from the surface may be used to determine the amount of absorption and/or scattering in the tissue in the regions where the narrow beams impinge. A region of large absorption may result in reflection of a small amount of light compared to a region of small absorption. A region of large scattering may result in reflection of a considerably broadened beam compared to a region of small sub-surface scattering. Soft or hard tissue in the ear may differ in the amount of light absorption and/or scattering. Thus this may be used to identify soft or hard regions in the ear.

In some embodiments the hardness under the skin is evaluated by detecting the reflected pattern of light, calculating the size of the regions of the pattern, and comparing this size with the regular or emitted size of the regions of the pattern.

In some embodiments the scanner comprises guide means adapted to assist the operator in holding the scanner in the correct distance and/or position relative to the ear.
The distance may be relative to an external part of the ear, the pinna, or relative to a part of the ear canal.

In some embodiments the guide means comprises two laser beams, and when the two laser beams are crossing each other at a point on the ear, the distance between scanner and ear is correct for scanning.
The point on the ear may a point on the external part of the ear.

In some embodiments the guide means comprises a first and a second set of two laser beams, and when the first set of laser beams are crossing each other at a first point on the ear, the distance between scanner and ear is correct for scanning of a first region of the ear, and when the second set of laser beams are crossing each other at a second point on the ear the distance between the scanner and ear is correct for scanning of a second region of the ear.
The points on the ear may be one point or two different points on the external part of the ear. The first and the second point may indicate the distance the scanner probe should be moved in/out of the ear.

The two sets of two laser beams may be of different color.

In some embodiments the ear is scanned in a first configuration where the mouth is closed, and in a second configuration, where the mouth is open.
It is an advantage because then the two extreme configurations of the jaw and mouth and thereby the ear are recorded.

Thus with regard to ear scanning it is possible to record a number of scans of one ear, and ask the patient to deliberately vary the size of the ear canal by swallowing, yawning, chewing, and drinking. In this way a series of scans of the ear canal can be recorded and the magnitude of the variation of the patient's ear canal can be detected.

In some embodiments the image data of the first and the second configurations are merged into one image by means of CAD.
Thus it is possible to superimpose the scans on one another to create a combined image or model, which will fit the patient's ear under all conditions. Such a model is naturally made as a combination of or a compromise between the different sizes of the ear canal.

In some embodiments a touch sensor is arranged in connection with the scanner, such that the touch sensor is adapted to register contact with the skin.

In some embodiments the touch sensor comprises a tactile component at the probe tip.
It is an advantage to also perform a touch or contact measurement to thereby obtain a double check of the real time image detection, and to acquire further data which can be used to obtain a complete scan of the ear canal.

In some embodiments the touch sensor is a capacitive sensor.
In some embodiments the direction of the ear canal towards the ear drum is determined by means of image data and/or one or more other data.
It is an advantage because when the direction of the ear canal towards the ear drum is known, the speaker or transceiver in a hearing aid can be arranged such that it points directly towards the ear drum, whereby the user of the hearing aid will receive the sounds directly at the ear drum, whereby the sound quality is improved for the user.

In some embodiments a motion sensor is arranged in connection with the scanner, such that the motion sensor is adapted to perform a motion measurement of the scanner.
It is an advantage also to perform motion measurement, since this can be used in registration.

In some embodiments an orientation sensor is arranged in connection with the scanner, such that the orientation sensor is adapted to perform an orientation measurement of the scanner.

In some embodiments the scanner comprises a number of marks in its cover, such that an operator can visually determine how far the scanner must be moved into the ear canal.
It is an advantage because when the scanner is moved into an ear canal, the operator may not be able to see the tip of the scanner. When the operator can see the marks of the scanner cover, he thereby has a guide to how far the scanner is moved into the ear, and when it should not be moved any further into the ear, in order not to be touching the ear drum. The marks may e.g. be three lines arranged with a distance from each other on the scanner cover.

In some embodiments a user interface of the scanner is adapted to direct the operator to insert the probe a certain distance into the ear canal.
It is an advantage since it may be used in registration.

In some embodiments a user interface of the scanner is adapted to direct the operator to move the probe a certain distance in any three-dimensional direction.
It is an advantage since it may be used in registration.

In some embodiments the shape of the probe prevents it from reaching the ear drum. It is an advantage because the ear drum or tympanic membrane is very sensitive. For example, the probe can have a conical longitudinal cross section, or a bulge.

In some embodiments a camera, such as a second camera or a video camera, is arranged in relation to the scanner such that it is configured to record images, from where the position of the scanner relative to the ear is adapted to be determined.
It is an advantage to record the scanner position relative to the ear by means of a regular camera capturing photographs or by means of a video camera.
In some embodiments the method comprises the steps of:
- varying the focus plane of the pattern over a range of focus plane positions while maintaining a fixed spatial relation of the camera and the surface,
- determining the in-focus position(s) of:
   a) each of a plurality of sensor elements in the camera for said range of focus plane positions, or
   b) each of a plurality of groups of the sensor elements in the camera for said range of focus planes, and
   transforming in-focus data into 3D real world coordinates.
The scanner may be integrated in a manageable housing, such as a handheld housing. One object is to discriminate out-of-focus information. A further object is to provide a fast scanning time.

The focus scanning may be achieved by a scanner (not claimed) for obtaining and/or measuring the 3D geometry of at least a part of the surface of an object, said scanner comprising:
- means for generating a probe light incorporating a pattern,
- at least one camera accommodating an array of sensor elements,
- means for transmitting the probe light towards the object thereby illuminating at least a part of the object with said pattern,
- means for imaging light reflected from the object to the camera,
- means for varying the position of the focus plane of the pattern on the object while maintaining a fixed spatial relation of the scanner and the object, and
- data processing means for:
   a) determining the in-focus position(s) of:
      - each of a plurality of the sensor elements for a range of focus plane positions, or
      - each of a plurality of groups of the sensor elements for a range of focus plane positions, and
   b) transforming in-focus data into 3D real world coordinates.

The method and apparatus relating to focus scanning is for providing a 3D surface registration of objects using light as a non-contact probing agent. The light is provided in the form of an illumination pattern to provide a light oscillation on the object. The variation / oscillation in the pattern may be spatial, e.g. a static checkerboard pattern, and/or it may be time varying, for example by moving a pattern across the object being scanned.
Focus scanning provides for a variation of the focus plane of the pattern over a range of focus plane positions while maintaining a fixed spatial relation of the scanner and the object. It does not mean that the scan must be provided with a fixed spatial relation of the scanner and the object, but merely that the focus plane can be varied (scanned) with a fixed spatial relation of the scanner and the object. This provides for a hand held scanner solution based on focus scanning.

Some embodiments comprises a first optical system, such as an arrangement of lenses, for transmitting the probe light towards the object and a second optical system for imaging light reflected from the object to the camera. In some embodiments only one optical system images the pattern onto the object and images the object, or at least a part of the object, onto the camera, e.g. along the same optical axis, however along opposite optical paths.

In some embodiments an optical system provides a confocal imaging of the pattern onto the object being probed and from the object being probed to the camera. The focus plane may be adjusted in such a way that the image of the pattern on the probed object is shifted along the optical axis, e.g. in equal steps from one end of the scanning region to the other. The probe light incorporating the pattern provides a pattern of light and darkness on the object. Specifically, when the pattern is varied in time for a fixed focus plane then the in-focus regions on the object will display an oscillating pattern of light and darkness. The out-of-focus regions will display smaller or no contrast in the light oscillations.

When a time varying pattern is applied a single sub-scan can be obtained by collecting a number of 2D images at different positions of the focus plane and at different instances of the pattern. As the focus plane coincides with the scan surface at a single pixel position, the pattern will be projected onto the surface point in-focus and with high contrast, thereby giving rise to a large variation, or amplitude, of the pixel value over time. For each pixel it is thus possible to identify individual settings of the focusing plane for which each pixel will be in focus. By using knowledge of the optical system used, it is possible to transform the contrast information vs. position of the focus plane into 3D surface information, on an individual pixel basis.

Thus, in one embodiment the focus position is calculated by determining the light oscillation amplitude for each of a plurality of sensor elements for a range of focus planes.

For a static pattern a single sub-scan can be obtained by collecting a number of 2D images at different positions of the focus plane. As the focus plane coincides with the scan surface, the pattern will be projected onto the surface point in-focus and with high contrast. The high contrast gives rise to a large spatial variation of the static pattern on the surface of the object, thereby providing a large variation, or amplitude, of the pixel values over a group of adjacent pixels. For each group of pixels it is thus possible to identify individual settings of the focusing plane for which each group of pixels will be in focus. By using knowledge of the optical system used, it is possible to transform the contrast information vs. position of the focus plane into 3D surface information, on an individual pixel group basis.

Thus, in one embodiment the focus position is calculated by determining the light oscillation amplitude for each of a plurality of groups of the sensor elements for a range of focus planes.

The 2D to 3D conversion of the image data can be performed in a number of ways known in the art. I.e. the 3D surface structure of the probed object can be determined by finding the plane corresponding to the maximum light oscillation amplitude for each sensor element, or for each group of sensor elements, in the camera's sensor array when recording the light amplitude for a range of different focus planes. Preferably, the focus plane is adjusted in equal steps from one end of the scanning region to the other. Preferably the focus plane can be moved in a range large enough to at least coincide with the surface of the object being scanned.

Handheld embodiments of the invention preferably include motion sensors such as accelerometers and/or gyros. Preferably, these micro electro mechanical systems (MEMS) should measure all motion in 3D, i.e., both translations and rotations for the three principal coordinate axes. The benefits are:
A) Motion sensors can detect vibrations and/or shaking. Scans such affected can be either discarded or corrected by use of image stabilization techniques.
B) Motion sensors can help with stitching/ registering partial scans to each other. This advantage is relevant when the field of view of the scanner is smaller than the object to be scanned. In this situation, the scanner is applied for small regions of the object (one at a time) that then are combined to obtain the full scan. In the ideal case, motion sensors can provide the required relative rigid-motion transformation between partial scans' local coordinates, because they measure the relative position of the scanning device in each partial scan. Motion sensors with limited accuracy can still provide a first guess for a software-based stitching/ registration of partial scans based on, e.g., the Iterative Closest Point class of algorithms, resulting in reduced computation time.
C) Motion sensors can be used (also) as a remote control for the software that accompanies the invention. Such software, for example, can be used to visualize the acquired scan. With the scanner device now acting as a remote control, the user can, for example, rotate and/or pan the view (by moving the remote control in the same way as the object on the computer screen should "move"). Especially in clinical application, such dual use of the handheld scanner is preferable out of hygienic considerations, because the operator avoids contamination from alternative, hand-operated input devices (touch screen, mouse, keyboard, etc).

The present invention relates to different aspects including the method described above and in the following, and corresponding apparatus, methods, devices, uses and/or product means, each yielding one or more of the benefits and advantages described in connection with the first mentioned aspect, and each having one or more embodiments corresponding to the embodiments described in connection with the first mentioned aspect and/or disclosed in the appended claims.

In particular, disclosed herein is a scanner (not claimed) for three-dimensional scanning of interior surfaces, comprising:
- at least one light source adapted to create and project structured light producing a pattern on the interior surface of an object,
- at least one camera, adapted to record 2D images of the pattern,
- data processing means adapted to convert 2D image information into 3D real world coordinates,
- the point of emission of light as well as the point of accumulation of reflected light for the camera being located on a probe having an axis,
- the at least one light source and the at least one camera being adapted to perform a scan around the axis,
- the probe being adapted to be entered into a cavity, and
wherein the scanner comprises:
- means for processing data such that surface information from areas of the surface, where image scanning is not complete, is created.

The probe of the scanner may be either rigid of flexible.
This scanner has the advantage that it may be able to cover the whole circumference without moving the scanner thus being able to scan the whole inner surface of an object. With this layout it is possible to scan interior surfaces such as the ear canal, tubes, pipes and bores with non-contact scanning and obtain high precision scan data of the whole interior surface of the object.

Furthermore, the dimensions of the scanner can be very small thus allowing scanning and 3D mapping of interior surfaces with small cross section, which are inaccessible to prior art scanners.

It is an advantage that the scanner is equipped with a position sensor, which allows the relative position and orientation of the scanner and the object to be determined for successive scans. This greatly facilitates the combination of data from successive scans and makes it possible to combine these with much higher precision irrespective of the position and orientation of the scanner during scanning.

The compact layout of the scanner allows for easy scanning of interior surfaces of objects of extremely small size. The ease of operation of the scanners according to the invention means that practitioners without experience in scanning can easily perform the scanning operations, which is required especially in the case of scanning of body cavities and scanning for archaeological purposes.

In some embodiments, the probe shaped scanner has an axis and the at least one light source and the at least one camera are adapted to perform a scan around the axis

In some embodiments, the data conversion device and said probe shaped scanner are integrated in one device.
In some embodiments, the data conversion device is part of a separate unit, such as part of a personal computer connected to the probe shaped scanner.

In some embodiments, the said data processing device and said probe shaped scanner are integrated in one device.

In some embodiments, the data processing device is part of a separate unit, such as part of a personal computer connected to said probe shaped scanner.

In some embodiments, the data processing device and said data conversion device are comprised in one integrated conversion and processing device. The integrated conversion and processing device and the probe shaped scanner may be integrated in one device

In some embodiments, the scanner system comprises a nontransitory computer readable medium having one or more computer instructions stored thereon, where said computer instructions comprises instructions for conversion of 2D images and/or for processing data.

Disclosed is a hearing aid device obtained by from a 3D model of the interior surface of an ear, where said 3D model is obtained by the method according to the invention and/or by the scanner according to the invention.

Disclosed is a nontransitory computer readable medium storing thereon a computer program, where said computer program is configured for causing computer-assisted scanning of partly obstructed interior surfaces.

Disclosed is a computer program product comprising program code means for causing a data processing system to perform the method described above when said program code means are executed on the data processing system, and a computer program product comprising a computer-readable medium having stored there on the program code means.

### Brief description of the drawings

The above and/or additional objects, features and advantages of the present invention, will be further elucidated by the following illustrative and nonlimiting detailed description of embodiments of the present invention, with reference to the appended drawings, wherein:
Figure 1 illustrates an embodiment of the interior surface scanner according to the invention.
Figure 2 shows a cross section of an embodiment of the interior surface scanner according to the invention.
Figure 3 illustrates another embodiment of the interior surface scanner with a mirror in front of the camera.
Figure 4 shows a cross section of another embodiment of the interior surface scanner with a mirror in front of the camera.
Figure 5 shows how a structured light pattern is projected onto the interior surface. In this case the pattern is a single cone. This pattern is then reflected from the surface into the camera.
Figure 6 illustrates an example of the use of mirrors and/or prisms. A structured light pattern is reflected in a mirror before being projected onto the interior surface. In this case the pattern is a single cone. This pattern is then reflected from the surface into a mirror that reflects the pattern into the camera.
Figure 7 shows a cross section of a model of the interior surface scanner according to the invention. Note that the camera has been moved out of the probe and a lens system is used to guide the image to the camera.
Figure 8 shows a cross section of a model of the interior surface scanner according to the invention. Note that the camera has been moved out of the probe and optical fibres are used to guide the image to the camera.
Figure 9 illustrates different positions sensors, which can be applied within the invention.
Figure 10 shows an embodiment of a hollow calibration object used for calibration of the camera and light sources. Note the symmetric 3D object feature curves on the object, which are utilised in the calibration.
Figure 11 shows a schematic sketch of a scanner according to the invention adapted for scanning of the ear and ear canal.
Figure 12 shows a schematic sketch of another embodiment of the scanner for the ear and ear canal.
Figure 13 shows a scan of an ear and an ear canal seen from two different views.
Figure 14 illustrates an embodiment of the scanner being able to scan the surface lying behind the end of the probe.
Fig. 15 shows an example of a scanner with a probe in an ear.
Fig. 16 shows an example of scanner with an extra camera mounted.
Fig. 17 shows examples of laser beams for guiding the position and orientation of the scanner.

In the following description, reference is made to the accompanying figures, which show by way of illustration how the invention may be practiced.

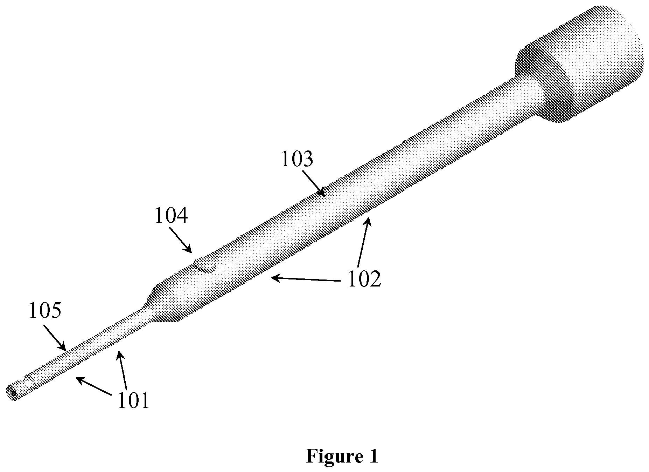

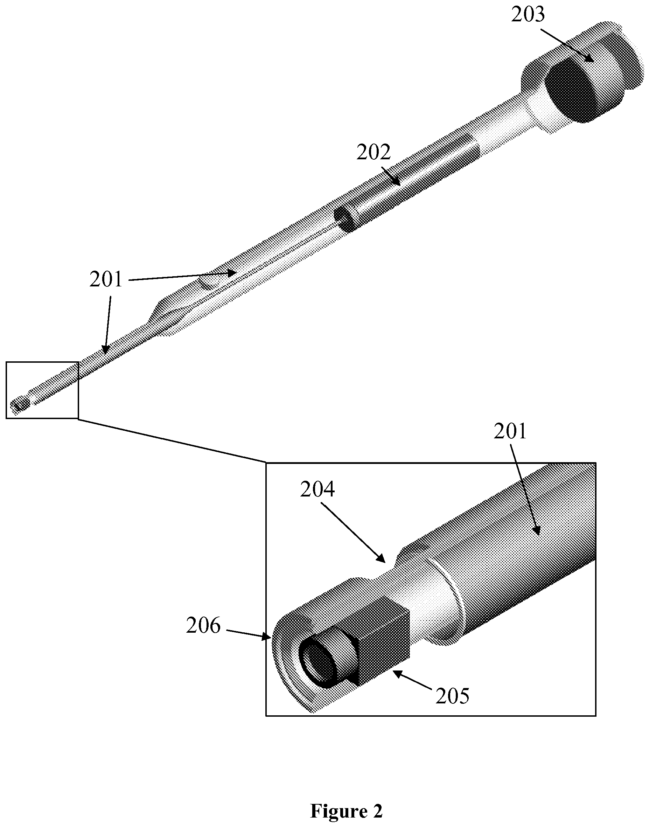

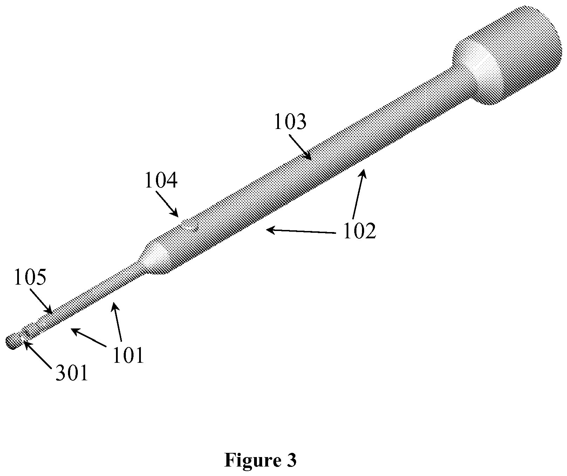

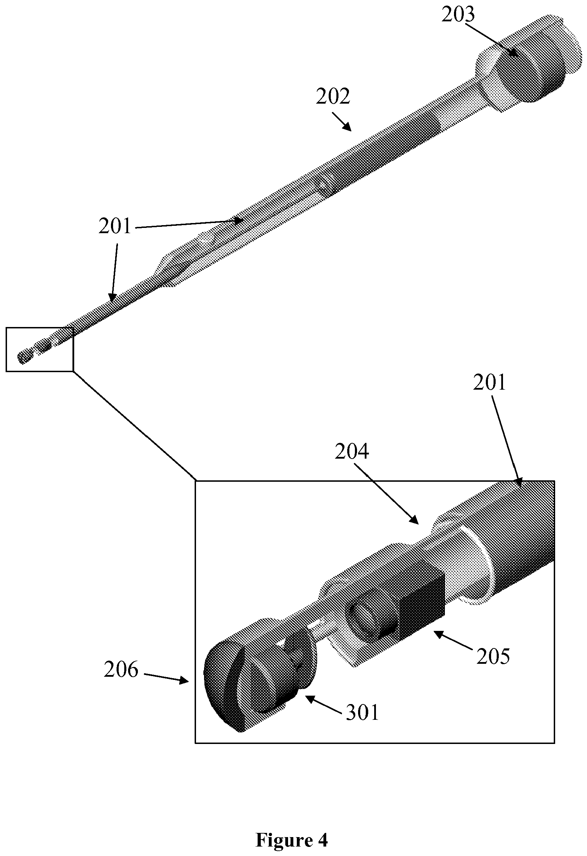

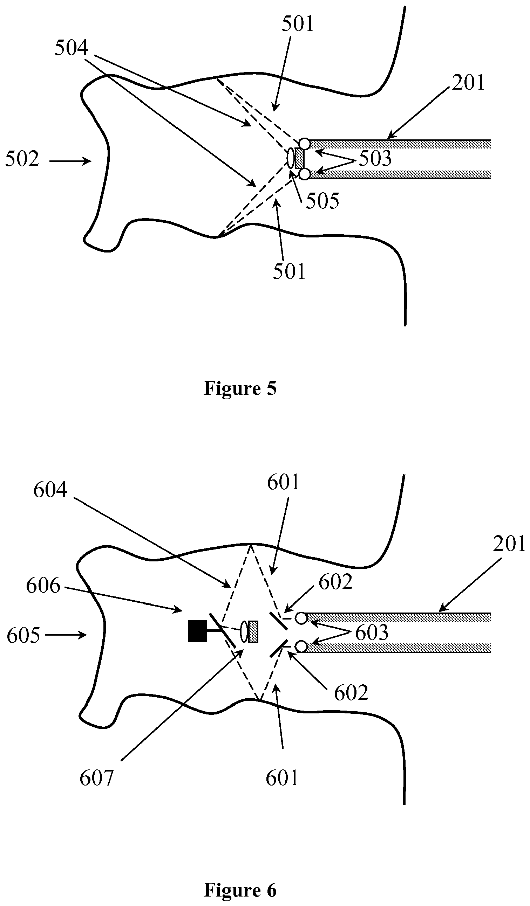

Figure 1 to Figure 4 illustrates two embodiments of the invention. The first part 101 of the scanner is the probe, which is inserted into the cavity. The second part 102 is a handle. The scanner in Figure 1 and Figure 2 comprises a cover 103, a scan button 104, a disposable cover 105, light guides 201, a light source 202, a position sensor 203, optics and mirrors and/or prisms 204, a camera 205 and a protector/collision detector 206. A rotating mirror and/or prism with a micro motor 301 is also added to the component list in the embodiment shown in Figure 3 and Figure 4. As illustrated in Figure 5, the scanner works by projecting a structured light pattern 501 onto the interior surface of the object 502. The camera 503 acquires images of the reflection 504 of the light pattern from the surface. By locating the light pattern in the images, the corresponding 3D surface positions can be reconstructed applying well-known projective geometry. The scanner only scans limited parts of the surface at each position and usually it has to be moved around handheld or automatically to scan the full interior surface.

The light is generated by one or more light sources such as lasers, variable output-powered laser, light emitting diodes (LED), halogen spots or other spotlights and travels through the light guides such as optical fibers. In some applications it might be relevant to use monochromatic, coherent or polarized light. At the end of the light guides optics and mirrors and/or prisms may create the desired pattern. Examples of optics are filters, lenses or prisms. An alternative to the use of light guides is to place the light source near the tip of the scanner. Note that the projection of light, even lasers, onto the surface does not damage the surface.

The light sources for some applications preferably are as small as possible to minimize the dimensions of the scanner. It is thus contemplated that the light source may have a cross section perpendicular to the direction of emitted light of less than 5 mm², preferably less than 4 mm², for example less than 3 mm², such as less than 2 mm², for example less than 1 mm², such as less than 0.5 mm², for example less than 0.25 mm².

The scanner may work with only one light source, but for many purposes it is advantageous to have several such as at least two light sources, such as at least three light sources, for example at least four light sources, such as at least five light sources, such as at least six light sources, for example at least seven light sources, such as at least eight light sources, for example at least ten light sources, such as at least twelve light sources, for example at least sixteen light sources, such as at least twenty light sources.

Depending on the desired pattern one, two, three or more optics and one, two, three, four or more mirror and/or prisms are required. The structured light pattern may be a number of rays forming a grid of spots on the surface consisting of one, two, three, four or more rows of points, one, two, three or more cones of light forming contours on the surface, one, two, three of more planes of light forming contours on the surface, one, two, three of more thick planes of light forming thick contours on the surface, a number of rectangular shaped rays forming a distorted checker board pattern on the surface or more complex shapes.

Thus, when projecting a pattern of rays, pattern may comprise at least 10 rays, such as at least 25 rays, for example at least 100 rays, such as at least 1000 rays, for example at least 10,000 rays, such as at least 100,000 rays, for example at least 1,000,000 rays.

Figure 5 illustrates how a single light cone 501 is projected onto the object surface 502 using optics 503. Figure 6 shows how the emission angle of the light cone can be increased significantly by reflecting the emitted light 601 into a cone mirror and/or prism 602 after the optics 603. Any type of mirrors such as coplanar mirrors and cone mirrors can be used to reflect the light. Applying mirrors and/or prisms make it possible to change the emission direction invariant of the orientation of the light guides. The light pattern can also be moved over the surface without moving the actual scanner by rotating and/or tilting the mirrors and/or prisms. The rotation and/or tilting of the mirrors and/or prisms may be carried out by a motor.

Preferably the location of the point of emission of light and the point of recording reflected light as well as the angle of emission and recording with respect to the axis of the probe are chosen to give an angle between incident light on the object and light reflected from the object of approximately 20-30 degrees. An example of this embodiment is illustrated in figure 6.

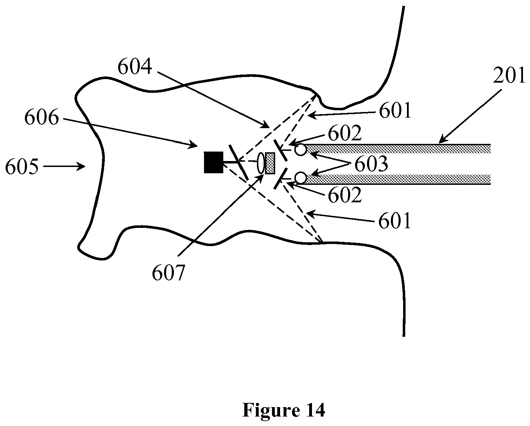

Occlusion effects represent a problem for some types of scanning of interior surfaces. Some of these can be overcome by selecting a direction of emission and recording of light with respect to the axis of the scanner, which ensures that light is projected on and recorded from all parts of the interior surfaces. One embodiment of the scanner is designed, wherein the location of the point of emission of light and the point of recording reflected light as well as the angle of emission and recording with respect to the axis of the probe are chosen to give a scan of the surface lying ahead of the end of the probe. An example of such a scanner is shown in Figure 5. Alternatively the location of the point of emission of light and the point of recording reflected light as well as the angle of emission and recording with respect to the axis of the probe may be chosen to give a scan of the surface lying approximately around the end of the probe. An example of this is shown in figure 6. Alternatively, the location of the point of emission of light and the point of recording reflected light as well as the angle of emission and recording with respect to the axis of the probe may be chosen to give a scan of the surface lying behind the end of the probe. Figure 14 illustrates an example of such a scanner. These alternative embodiments may be obtained with one scanner by tilting mirrors and/or prisms.

The light source intensities are preferably varied depending on the surface and color of the object to be scanned. Preferably the intensity should be determined automatically using automatic light source intensity calibration.

The intensity calibration may be performed by inserting the scanner into the object and calculate a number of histograms from the acquired images. First a histogram is calculated with the light source turned off. A second histogram is the calculated when the light source is turned on with an arbitrary intensity. The first histogram is then subtracted from the second to remove the background intensity. The intensity is then adjusted until the requested quantile corresponds to a predefined intensity. The background could also be removed by subtracting the image corresponding to the light source turned off from the image with light. The histogram used to determine the intensity could then be calculated from this difference image.

The images are acquired by the one or more cameras. Preferably the cameras comprise a lens and a sensor array such as a CCD or CMOS chip. Usually the camera also comprises a filter placed in front of the sensor array. The effect of the filter is that only light with approximately the desired wavelength passes the filter. This makes it feasible to separate different light sources in the scanner and remove most of the background light. Alternatively, the camera may be color sensitive.

The scanner may comprise just one camera or comprise several such as at least 2 cameras, such as at least 3 cameras, for example at least 4 cameras, such as at least 6 cameras, for example at least 7 cameras, such as at least 8 cameras, for example at least 10 cameras, such as at least 12 cameras, for example at least 16 cameras, such as at least 20 cameras.

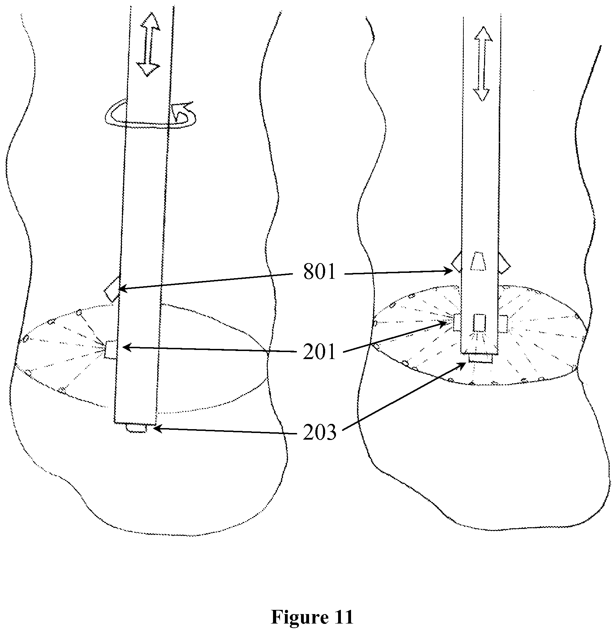

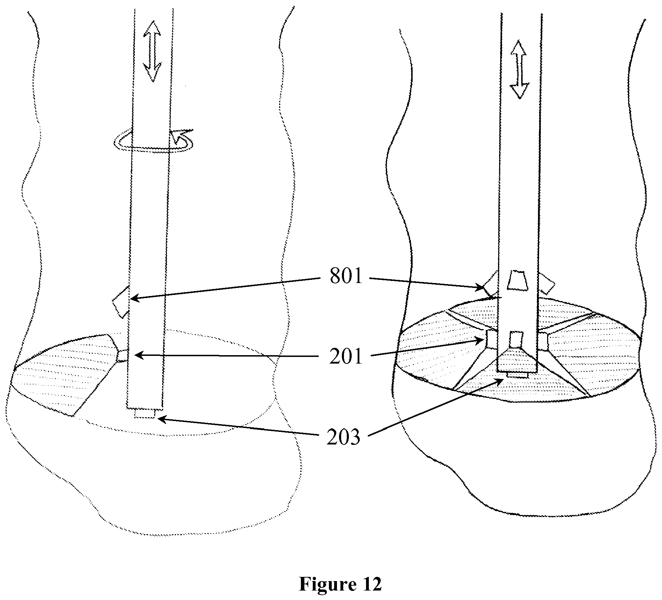

Preferably the cameras are arranged such that reflected light is recorded from different directions covering the 360 degrees around the probe, as seen in Figures 11 and 12, right side.

Preferably the camera part of the scanner is as small as possible. The size of commercially available cameras is reduced almost every year, and it is estimated that the lower limit for camera size and pixel size have not been reached at all yet. Irrespective of the future development within this area, any camera smaller than the cameras presently available will be suitable for use in the present invention. Therefore the light detecting component of the camera may have a cross section in a direction perpendicular to the direction of incident light of less than 10 mm², such as less than 9 mm², for example less than 8 mm², such as less than 7 mm², for example less than 6 mm², such as less than 5 mm², for example less than 4 mm², such as less than 3 mm², for example less than 1 mm², such as less than 0.5 mm², for example less than 0.25 mm², such as less than 0.1 mm², for example less than 0.01 mm².

The number of pixels of the camera is a question of the size of the camera, depending on the size of the pixels, the computing power used for processing the results of the scans and the cost of the camera. No upper limit for the number of pixels can be set, since precision is increased whenever the number of pixels is increased. Accordingly, the camera may comprise an array of at least 125x125 pixels, more preferably at least 250x250 pixels, more preferably more than 500x500 pixels, more preferably more than 1000x1000 pixels, such as more than 2000x2000 pixels, for example more than 4000x4000 pixels, such as more than 8000x8000 pixels, for example more than 10,000x10,000 pixels, such as more than 25,000x25,000 pixels, for example more than 50,000x50,000 pixels, such as more than 100,000x100,000 pixels, for example more than 250,000x250,000 pixels, such as more than 500,000x500,000 pixels, for example more than 1,000,000x1,000,000 pixels. Similarly, the pixel size may be the smallest available on the market, for example wherein a cross section of a pixel is less than 100 micrometres, such as less than 50 micrometres, for example less than 25 micrometres, such as less than 20 micrometres, for example less than 15 micrometres, such as less than 10 micrometres, for example less than 7.5 micrometres, such as less than 5 micrometres, for example less than 2.5 micrometres, such as less than 2 micrometres, for example less than 1.5 micrometres, such as less than 1 micrometres, for example less than 0.5 µ, such as less than 0.25 micrometres, for example less than 0.1 micrometres, such as less than 0.01 micrometres.

The light pattern may be reflected from the surface directly into the camera or into one or more light reflecting means such as mirrors or prisms before ending up in the camera. In the embodiment of the scanner in Figure 1 no mirrors are applied, since the scanner only needs to "look" forward with respect to the camera, i.e. the direction of the view is always parallel with the optical axis of the camera. Figure 5 illustrates the simple emission of the light pattern 501 and its reflections 504 from the object surface 502 into the camera 505 without the use of mirrors. Figure 5 is a simplified illustration of the principle used in the scanner in Figure 1.

Applying one or more mirrors and/or prisms for reflecting the light into the camera gives full freedom to select the direction of the view invariant of the orientation of the camera. Figure 6 illustrates how the emitted light pattern 601 is reflected using a cone mirror and/or prism 602 before it hits the object surface 605. The reflected light 604 is likewise reflected into a mirror and/or prism 606 before entering the camera 607. Figure 6 is a simplified illustration of the principle used in the scanner in Figure 3. Static mirrors such as coplanar or cone mirrors can be applied directly in the invention. Static mirrors have the advantage of being simple and mechanically stable.

In the embodiment of the scanner shown in Figure 3 the mirror and/or prism in front of the camera is coplanar, circular and able to rotate. The advantage of a rotating mirror and/or prism compared to a static mirror and/or prism, such as a cone mirror and/or prism, is that the image resolution and the field of view of the camera are significantly increased. Indeed resolution and field of view are seriously limited due to the small dimensions of the scanner, which directly affect the accuracy and flexibility. Tilting the mirror and/or prism further increases the accuracy and flexibility. In practice, the same mirror and/or prism can be used to generate the light pattern and reflecting the light into the camera. However, applying different mirrors and/or prisms for light and cameras, as presented in Figure 3, increase the flexibility of the scanner especially with respect to direction of view, depth of field and point reconstruction quality.

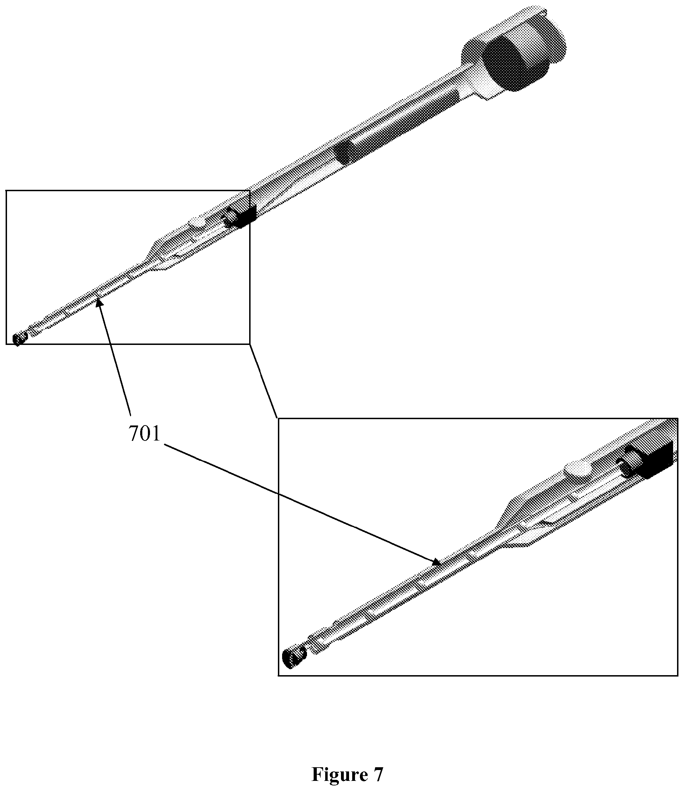

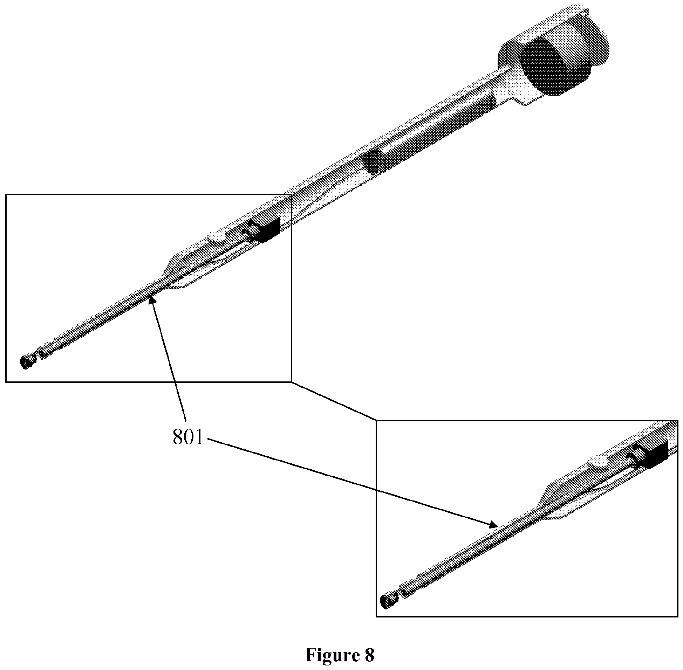

In the case of very small dimensions of the cavity and/or high requirements for accuracy it is infeasible to place the camera on the head of the scanner. The problem is solved by moving the cameras out of the probe. The image/light is then directed into the cameras by the use of light guides such as a lens system or optical fibres. An embodiment of the invention where a lens system 701 and optical fibres 801 are used as light guides are illustrated in Figure 7 and Figure 8, respectively. The lens system might be similar to the lens systems used in periscopes and endoscopes. At the moment the lens system is superior to optical fibres with respect to smallest dimensions and image quality. The disadvantage of the lens system is that it requires the probe be rigid, whereas the optical fibres are fully flexible, i.e. the probe can be flexible.

The objective of the position sensor is to determine the relative position and orientation of the probe head with respect to the object to be scanned. Knowing this position is extremely advantageous in combining the individual scans when the scanner or object is moved. Errors in the position measures will directly affect the quality of the scan. In the case of non-fixed objects such as the ear canal of humans are scanned, it is extremely advantageous to measure the position with respect to the object, e.g. the ear canal, and not to a fixed coordinate system, since the object might move during the scanning.

The position sensor is only used to combine the individual scans. The position sensor can be rendered superfluous by a registration of the individual scans. The output of the registration is the relative position of the scans. Knowing the relative positions of the scans makes it straightforward to combine the scans. For the registration to be successful the interior surface needs to contain a proper number of distinct features, which is not always the case.

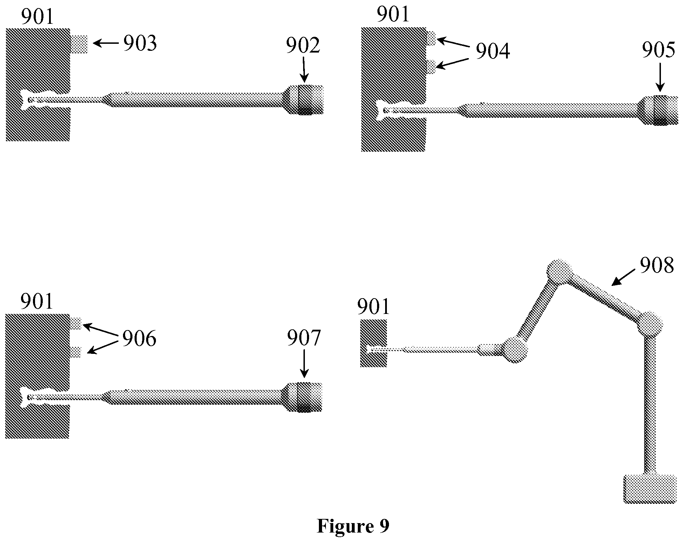

The position sensor can be a magnetic sensor as shown in Figure 9, where the receiver 902 usually is in the scanner and the transmitter 903 is secured to the object 901, e.g. the head of a human. Magnetic sensors have the advantage of not suffering for occlusion problems. Alternative sensors might be optical or sonic sensors. Figure 9 illustrates an optical sensor where markers 904 are placed on the object and a sensor 905 on the scanner. Likewise Figure 9 illustrates a sonic sensor, where an emitter 906 is placed on the object and a detector 907 is placed on the scanner. Both optical and sonic sensors suffer from occlusion problems, but their cost is often lower and the precision superior to those of magnetic sensors. In the case of a fixed object or an object, which can be fixed, a mechanical position sensor becomes attractive. As illustrated in Figure 9 these sensors usually consist of a number of joints 908 connected by encoders. Many mechanical sensors are highly accurate, but they tend to be bulky or cumbersome to use.

In general, the position needs to be determined with respect to the head of the scanner. More precisely, the position of the focal point of the camera has to be determined when the camera is placed on the probe head. In the case where light guides are used in front of the camera, the position should correspond to the tip of the guides. With a rigid design of the scanner cover as in Figure 1 to Figure 4 the position sensor can be placed anywhere on the scanner, since the relative distance between the scan head and the position sensor is constant. With a flexible design of the probe the position sensor needs to be placed on the scan head, e.g. at the front as on the scanner in Figure 11 and Figure 12.

In the design of the scanner show in Figure 1 and Figure 3 only the probe 101 is supposed to move into the cavity. The main objective of the design has been to minimize the width of this part, since it determines the minimal size of the cavity, which can be scanned. In general the width of the probe can be varied freely down to approximately 0.1 mm, e.g. the width can be 30, 20, 15, 10, 8, 6, 5, 4, 3, 2, 1 or 0.1 mm. However the final design is a trade-off between size, accuracy and mechanical stability. In general the application determines the desirable design.

In the case of scanning the human ear canal the width of the part is requested to be below 4 mm. Figure 3 shows a scanner designed for scanning ear canals, where the width of the probe is 3.5 mm. The length of the probe can also be varied freely down to approximately 5 mm, e.g. the length can be 20, 35, 50, 100, 200, 300 or 500 mm. The length of the probe shown in Figure 1 and Figure 3 is 55 mm.

The rest of the scanner's cover is basically a handle. For optimal handling this part should preferably be 10-30 mm width and 100-150 mm long. The dimension can however be varied freely. As in Figure 1 and Figure 3 the width of the handle may be extended to make room for the components, e.g. the position sensor. The dimensions of this extension should however be minimized if the objective is to create the smallest and lightest scanner. The width and length of the extension shown in Figure 1 and Figure 3 is 40 mm and 30 mm, respectively. Note that larger light sources such as halogen spots may be moved to the extension.

In another embodiment of the scanner it is possible to rotate the probe around its axis. The advantage of this design compared to only rotating the mirrors and/or prisms as in Figure 3 is that the motor can be placed in the handle. Likewise another embodiment comprises a linear drive, which is able to translate the probe along its axis. The scanner can also be mounted on a robot, or another device, which is able to position the scanner with any orientation and position within its workspace.

The choice of material for the cover depends on the actual application, especially whether the probe needs to be rigid or flexible. Preferably the cover should be produced in stainless steel or from a material selected from a group consisting of alloy, aluminum, a plastic polymer, Kevlar (R), ceramics or carbon.

In some application it might be necessary to protect the components such as cameras, mirrors and/or prisms and lenses against dust and other dirt. In practice this is done by inserting a window of transparent material such as glass or a plastic polymer in the holes in front of the relevant components.

Other features in the preferred embodiment are a protector/collision detector, a scan button, and a disposable scanner cover. The protector consists of soft material such as rubber, silicone or a plastic polymer and ensures that the tip of the probe and the surface are not damaged in the case of a collision. In the case of scanning an ear canal it is crucial that the scanner does not damage the eardrum. In the case of very fragile surfaces, a collision detector adapted to measure the distance from the tip of the scanner to the bottom of the interior surface is added to the protector. When surfaces are scanned for which the scanner is subject to hygiene requirements, a disposable cover is desirable to minimize the need for cleaning. The disposable cover will usually only cover the probe or parts of it, but can be fit to the specific requirements. The scan button is used to start and stop the scan operation.

The acquired images may be analyzed real-time in a digital signal processor or microprocessor, which is placed in the scanner handle or in a separate processing box. The first step in the analysis of an image may be to detect the light pattern in the image using a standard tracking algorithm. When the light pattern is known, potentially with sub-pixel precision, the corresponding 3D coordinates can be reconstructed using well-known projective geometry. A precise reconstruction of the 3D coordinates requires a very high quality of the camera and light calibration. The next step may be to combine the 3D coordinates from different images acquired at the same or at different positions. The merging may simply be performed by combining the individual points sets positioned with respect to their relative position. Finally the points may be triangulated using a standard triangulation algorithm to form the final surface of the 3D model. The 3D model may then be transferred over a network to the destination for further use.

The scanner according to the invention is especially adapted for scanning interior surfaces, such as body cavities and other interior surfaces with narrow openings, into which light from an external scanner cannot enter due to occlusion effects.

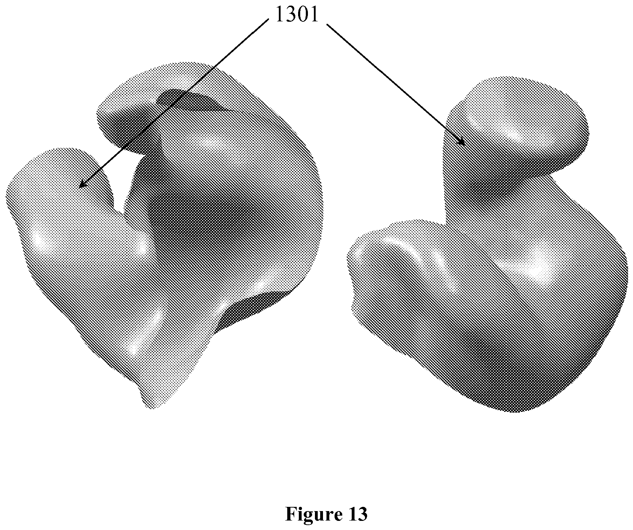

It is thus envisaged that the scanner is advantageous for scanning body cavities such as the internal surfaces of the ear, nose, mouth, teeth, stomach, lungs, alveoli, throat, rectum, vagina, veins, blood vessels, urinary tract. Scanning of teeth can be used in connection with correction of teeth and manufacture of dental implants. Scanning the blood vessels may be useful in connection with surgery. Scanning the vagina can be used in connection with pregnancy and delivery and also for measuring and modeling an individually adapted diaphragm. Figure 13 shows a scan of the interior surface of an ear and an ear canal 1301.

The scanner can also be used for industrial purposes such as for scanning internal surfaces of engines, fuel canals, bore, internal thread, pipes, tubes and containers. In this way the exact dimensions, such as volume and/or cross section and/or location of features, of the devices can be measured. When using a scanner with a position sensor, this can be done more precisely than with any of the known scanners. Furthermore, the present scanners are not sensitive to small deviations in the orientation of the axis of the scanner with respect to the axis of the object being scanned.

Another use is for archaeological purposes such as for scanning internal surfaces of jars, skulls and other archaeological items.

Furthermore, the scanners are very useful in industrial design especially in connection with computer assisted 3D modeling.

A possibility according to the invention is to scan the ear canal directly. This can be done by building the components of the scanner into an apparatus, which can be inserted into the ear of the patient. Embodiments of this scanner are shown in Figure 11. Preferably, the light source, e.g. the laser, and the camera are located outside the ear. The laser light can be carried into the scanner by light guides 201, and similarly, the reflected signals can be carried to a camera by another light guide 801. The scanner also consists of a position sensor 203, which measures the relative position of the scanner with respect to the object. During the scan, the scanner preferably rests on the edge of the ear canal, most preferably in those places where bones are closest to the skin surface. This is in order to obtain the highest stability and is very important, since the scanner itself works with an accuracy of less than 0.05 mm. The length of the ear canal can be scanned by moving the scanner in or out and record a series of overlapping images of the ear canal. The scanner may comprise only one laser source and one camera as the one shown in the left of Figure 11. In that case the scanner has to rotate while the camera records images. The scanner may comprise multiple laser sources such as four as shown in the scanner in the right part of Figure 11. The presence of multiple laser sources and cameras removes the need for rotation of the scanner in the ear canal. In the laser scanner disclosed in Figure 11, the laser source or sources project a ray of laser light on the surface of the ear canal.

Another type of ear canal laser scanner is shown in Figure 12. Here the laser light is projected as laser sheets producing a laser contour on the surface of the ear canal. Thereby, more rapid scanning can be performed compared to the above laser scanner. In the scanner shown in the right part of Figure 12, four laser sheets and four cameras are present. Thereby the laser sheets cover the whole circumference and rotation of the scanner is not required.

The same types of variation of the ear canal scanner can be used as in other cases of three-dimensional scanners according to this invention. Thus, the scanner may comprise at least two cameras, such as 4 cameras, such as for example 6 cameras. Likewise, there may be several laser sources such as for example 2 lasers creating laser sheets with an offset of 180 degrees, such as 3 laser sheets with an offset of 120 degrees, or 4 laser sheets with an offset of 90 degrees.

Currently hearing aids are created in a silicon mould, made with an ear impression.

It is possible to scan and create very detailed and accurate copies of ear impressions with the developed system as described in 3Shape's prior art document "Method for modeling customized earpieces" with publication number WO02071794 published in 2002.

Apart from hearing aids, other devices could also be inserted into a shell made to fit the ear canal of an individual. Such devices that could advantageously be incorporated into a shell manufactured according to the disclosed method include mobile phones, communication devices, loud speakers, tinnitus masking devices, or devices recording vibrations in the skull and transforming these into an audio signal.

Devices that may be incorporated into a shell in the ear also comprise devices related to Man Machine Interface (MMI) products, such as custom made ear microphone or receivers that enable reliable and clear communication even in the noisiest environments, or products related to wireless internet applications.

Speech not only creates sound waves, it also generates vibrations within the skull of the speaker. These vibrations can be picked up in the ear, but they may be picked up other places too, but by far the most convenient method is to do it in the ear. In one piece, a device thus may comprise a microphone to pick up the speech of the person wearing it, and a loudspeaker to transmit the speech of the communication partner. It is important that such devices are made to fit the ear.

Devices based on detection of vibration instead of sound can be used in the noisiest environments, since they only pick up the speech of the wearer and they allow for quiet communication, since the speaker can speak with a low voice when needed. The devices allow for completely hand-free communication.

Such a device is naturally also devoid of any kind of acoustic feedback if manufactured using the present invention.

The precision of the light sources and cameras is very high today and so is that of the software developed to detect the intersection of the light sheet with the object and to convert the two-dimensional data to three-dimensional coordinates. Therefore differences in precision and hence improvement of the precision primarily resides in the calibration of the systems. Recall that precision is of utmost importance in many applications.

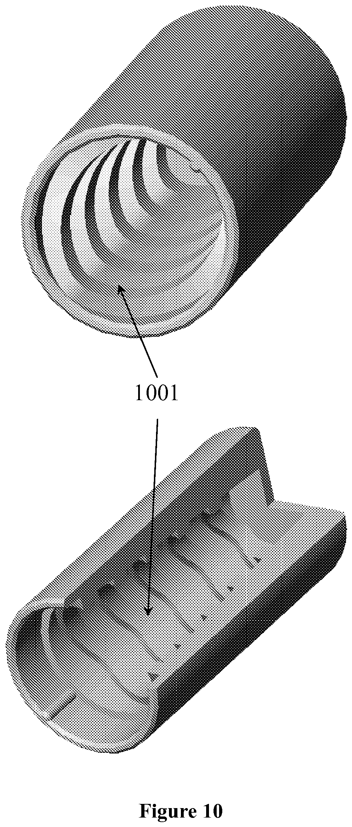

To obtain the highest precision both the light pattern and the camera need to be calibrated. Preferably the calibration should be performed using a calibration object with symmetrical 3D object feature curves and the corresponding methods as described below and in 3Shape's prior art document "Method for modeling customized earpieces" with publication number WO02071794 published in 2002. The main advantage of this type of calibration objects is that the light pattern can be calibrated independently of the calibration of the camera. An embodiment of the hollow calibration object used for calibration of the scanner is shown in Figure 10. Note the symmetric 3D object feature curves 1001 on the calibration object, which are utilized in the calibration.

Preferably, a light pattern is projected onto the calibration object to produce 2D image feature curves in the acquired images.

When preferred, the image feature curves may be determined using the Hough transformation, filter search, max intensity, threshold, center of gravity, derivatives or other procedures.

The image feature coordinates are found as the intersection between image feature curves. These intersections could be seen in the images as corners or sharp edges of the image feature curves. The image feature coordinates may be found as the intersection between the image feature curves such as the intersection between two n^{th} order curves, as the intersection between two first order curves, as the intersection between two second order curves, as the intersection between two third order curves, as the intersection between a fist order curve and a second order curve, as the intersection between a first order curve and a third order curve, or as the intersection between a second order curve and a third order curve or as the intersection between any other possible combination of curves.

Preferably, the calibration method comprises plotting of a mathematical combination of image feature points or features derived from these points against the angle of rotation or the translation of the calibration object. By plotting this function and optionally estimating a mathematical function describing the relationship between the function of an image coordinate and the angle of rotation or the translation, estimation of the light parameters and angle or rotation and/or translation becomes especially precise. The method may comprise determination of the mean plane of symmetry in the plot.

The mean plane of symmetry can be determined by calculating the mean angle of rotation / mean translation for pairs of image feature points having the same value in the mathematical combination. Doing this produces multiple estimates for the encoder offset and light pattern displacement allowing also for the estimate of the laser sheet angle.

Light pattern calibration may also comprise selecting symmetric points, plotting of the rotation angle and/or the translation for the first point against the difference in the rotation angle and/or the translation between the two symmetric points, deriving a mathematical formula for the plotted lines and estimating the light pattern parameters.

Alternatively, mathematical formulas can be derived for the curves that appear in some of the plots of the mathematical combination as a function of the angle of rotation or the translation. Given these curves and the corresponding formulas, the encoder offset, the light pattern displacement, and the light pattern angle can be estimated.

Preferably, light pattern coordinates of the 3D object feature curves are estimated corresponding to a discrete number of values of angle of rotation and/or translations. These values should preferably cover the whole circumference and/or length of the calibration object.

2D coordinates of the 3D object feature curves corresponding to a discrete number of values of angle or rotation and/or translation may be calculated from mathematical functions determining the 3D object feature curves. In order to determine the calibration parameters such as camera position, camera orientation, and camera optic parameters, pairs of 2D light pattern coordinates are compared to calculated 2D coordinates for a discrete number of values of angle or rotation and/or translation. This comparison preferably comprises using the Tsai or the Heikkilae algorithm. The advantage of the Tsai and the Heikkilae algorithms in this context is that they provide rapid and precise estimation of the calibration parameters such as radial lens distortion.

Alternative methods for calibration comprise direct linear transformation and direct non-linear matrix transformation optionally in combination with an optimization procedure such as least squares to minimize the error. In these cases initial calibration parameters may be estimated to facilitate the convergence of the parameters during optimization.

To improve calibration, precision outliers may be excluded from the calibration. Outliers can e.g. be identified in the plot of the mathematical combination of image feature coordinates against the angle of rotation / the translation or by back projection of coordinates after an initial calibration.

Two percent of the feature points deviating most from the back-projected 2D image feature curves may be excluded from the calibration or at least 3 percent, such as at least 5 percent, for example at least 10 percent, for example at least 12 percent, such as at least 15 percent for example at least 20, preferably at least 25 percent, for example at least 30 percent, more preferably at least 33 percent may be excluded to improve calibration precision.

In order to cover the whole circumference of the calibration object the discrete number of values for angle of rotation / translation may be at least 100, preferably at least 240, for example at least 500, such as at least 750, for example at least 1000, such as at least 1200, for example at least 1500, such as at least 1800, for example at least 2000, such as at least 2400, for example at least 3000, for example at least 3600, such as at least 4200. The higher the discrete number of values of angle of rotation / translation, the higher the calibration precision.

The highest calibration precision is obtained when using a rigid setup, which comprises mounting the calibration object onto mounting means.

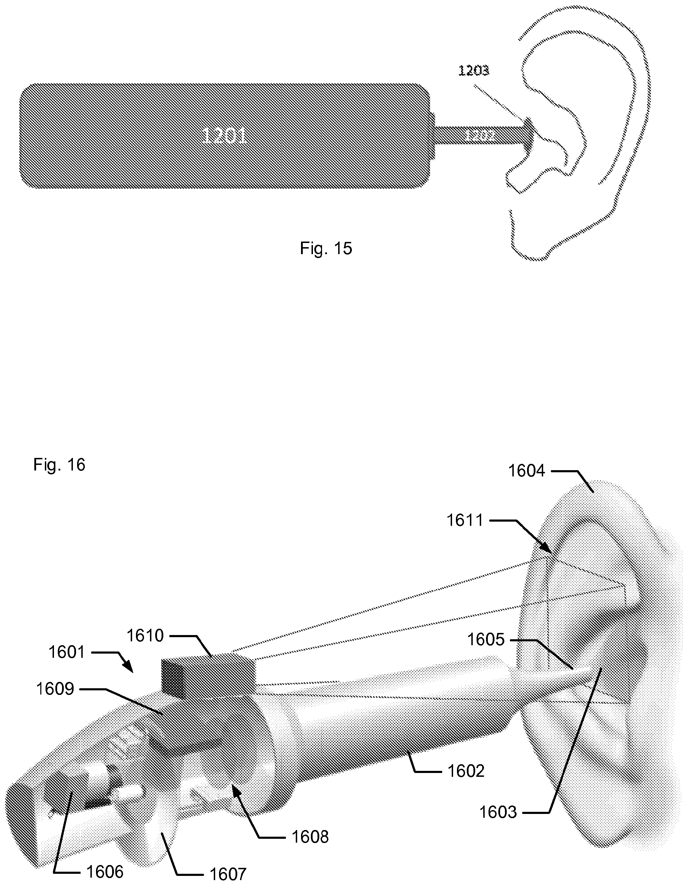

Fig. 15 shows an example of a scanner with a probe in an ear.

The scanner 1201 comprises a probe 1202, where the tip of the probe 1202 is shown to be partly inserted in the ear canal of a patient. Light 1203 is emitted from the probe tip of the scanner 1201 in order to scan the ear and/or the ear canal.

Fig. 16 shows an example of scanner with an extra camera mounted.
The scanner 1601 is arranged such that the ear 1604 and/or ear canal of a patient can be scanned. The scanner 1601 comprises a probe 1602, which comprises a probe tip 1605 from where light 1603 is emitted from the scanner.
The scanner 1601 is shown with a cut-off section such that the components inside the scanner 1601 can be viewed. The scanner comprises a light source 1606 which emits light 1603, a pattern 1607 which produces structured light from the light source 1606, a beam splitter (not shown), optical lenses 1608, and a camera or image sensor 1609 for capturing optical images. On the external portion of the scanner 1601 a camera 1610 is arranged, which is adapted to record images, for example as still photographs or video. The field of view 1611 of the camera 1610 is shown to be in the direction of the probe tip, such that the camera 1610 records images of, for example, the external part of the ear 1604. These recordings may be used as reference images for detecting the position and/or orientation of the scanner for example for coupling the scanned 2D images to a 3D position, and from this the position of the scanner 1601 relative to the ear 1604 is adapted to be determined.

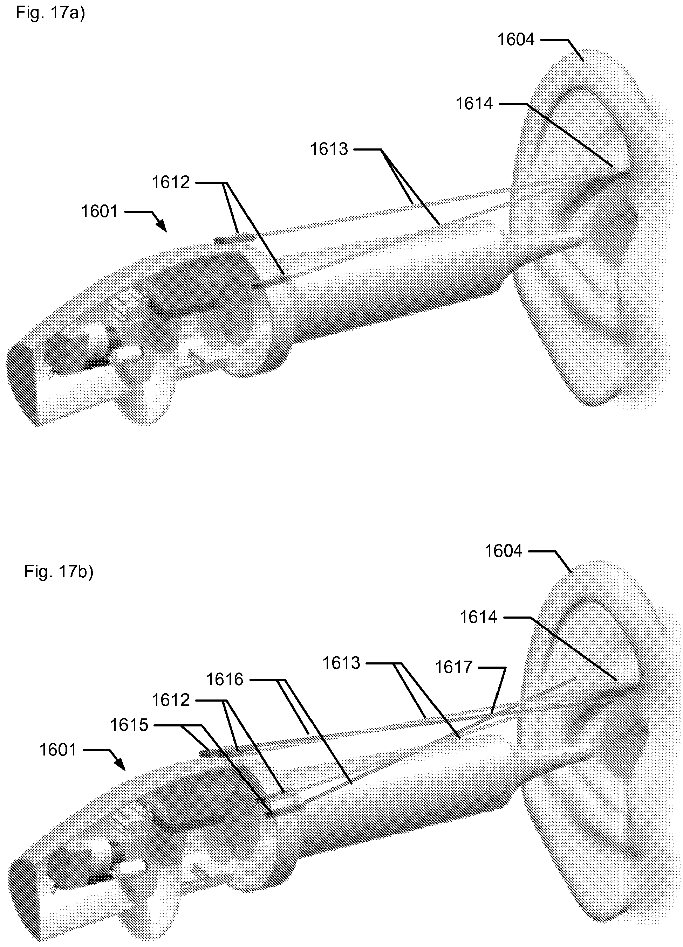

Fig. 17 shows examples of laser beams for guiding the position and orientation of the scanner.
Both fig. 17a) and fig. 17b) shows the scanner 1601 and the ear 1604.
In fig. 17a) two laser beams 1613 from two light sources 1612 arranged on the external part of the scanner 1601 guide the scanner operator (not shown) to hold the scanner 1601 in the correct distance from the ear 1604 for scanning. The laser beams 1613 cross each other at a point 1614, and for example when this point 1614 is at the external surface of the ear, then the scanner 1601 is in the correct distance from the ear 1604.

In fig. 17b) two sets of two laser beams 1613, 1616 from two sets of light sources 1612, 1615, respectively, guide the scanner operator (not shown) to hold the scanner 1601 in the correct distance from the ear 1604 for scanning. The two sets of light sources 1612, 1615 are arranged on the external part of the scanner 1601. The two laser beams 1613 cross each other at a first point 1614, and the two other laser beams 1616 cross each other at a second point 1617. When for example the first point 1614 is at the external surface of the ear, then the scanner 1601 is in a correct first distance from the ear 1604, and when for example the second point 1617 is at the external surface of the ear, then the scanner 1601 is in a correct second distance from the ear 1604. The first distance may be the distance in which a first set of scanning images are made, and afterwards the scanner 1601 may be moved into the ear to the second distance to capture a second set of scanning images or vice versa such that the scanner 1601 is first arranged at the second distance to capture images and then the scanner is moved out of the ear to the first distance to capture images there.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

The features of the method described above and in the following may be implemented in software and carried out on a data processing system or other processing means caused by the execution of computer-executable instructions. The instructions may be program code means loaded in a memory, such as a RAM, from a storage medium or from another computer via a computer network. Alternatively, the described features may be implemented by hardwired circuitry instead of software or in combination with software.

### References

[1]: TSai, R. Y., "A versatile Camera Calibration Technique for High-Accuracy 3D Machine Vision Metrology Using Off-the-Shelf TV Cameras and Lenses", IEEE Journal of Robotics and Automation, pages 323-344, Vol. RA-3, No. 4, August 1987.
[2]: Heikkilae, J, "Geometric Camera Calibration Using Circular Control Points", IEEE Transactions on Pattern Analysis and Machine Intelligence, Vol. 22, No. 10, pp. 1066-1077, Oct 2000.
[3]: Wang et al., "Filling holes on locally smooth surfaces reconstructed from point clouds". Image and Vision Computing Vo. 25 (2007) pp. 103-113.

## Claims

1. A method for scanning partly obstructed interior surfaces, where the method comprises:
- providing a probe shaped scanner (101, 102, 1201) having an axis, where the probe shaped scanner comprises:
• at least one light source (202) configured to create and project structured light, and
• at least one camera (205, 505) configured to record 2D images;
- entering said probe shaped scanner into a cavity of an object (502), where said cavity is bounded by an interior surface (502) of the object;
- creating and projecting structured light (501) from said light source of the probe producing a pattern on the interior surface of the object;
- recording a series of 2D images of the reflection of the pattern from the interior surface using said camera;
- combining said series of 2D images to obtain 3D real world coordinates of the interior surface;
- registering one or more holes in the 3D real world coordinates corresponding to areas of the interior surface where visual access is blocked due to the geometrical form of the interior surface; and
- providing data and processing said data to create 3D real world coordinates for the areas of the interior surface where said visual access is blocked.

2. The method for scanning according to claim 1, wherein part of the data of surface information of the left ear is used as a part of the data of surface information for the right ear, if there are parts of the right ear where surface information has not been acquired, and vice versa.

3. The method for scanning according to any of the preceding claims, wherein holes in a scan are inferred or interpolated based on previous scans of the person's ear.

4. The method for scanning according to the preceding claim, wherein the method comprises:
- providing that the camera (205, 505) comprises a plurality of sensor elements or a plurality of groups of sensor elements;
- varying the focus plane of the pattern over a range of focus plane positions while maintaining a substantially fixed spatial relation of the camera and said interior surface,
where the combining of 2D images comprises determining by analysis of said 2D images the in-focus positions of the focus plane for:
a) each of a plurality of sensor elements in the camera for said range of focus plane positions, or
b) each of a plurality of groups of the sensor elements in the camera for said range of focus planes,
and where said 3D real world coordinates are from said in-focus positions.

5. The method for scanning according to any of the preceding claims, wherein holes in the surface information are closed by fitting to higher order mathematical surfaces, such as second order, third orders, fourth order etc.

6. The method for scanning according to any of the preceding claims, wherein holes in the surface information are closed by using information about where there exists no surface.

7. The method for scanning according to any of the preceding claims, wherein holes in the surface information are closed by using other data than image data, such as color, interference, angle of reflected light, and/or data from one or more other sensors than the camera.

8. The method for scanning according to any of the preceding claims, wherein foreign objects in the ear canal are identified.

9. The method for scanning according to claim 8, wherein identifying foreign objects comprises detecting reflections from the foreign object, and analyzing the difference in the reflected light from the skin and the foreign object, respectively.

10. The method for scanning according to any of the preceding claims, wherein the method comprises using monochromatic light to illuminate the surface for analyzing reflected light.

11. The method for scanning according to any of claims 8 to 10, wherein the method comprises identifying foreign objects by correlating surface data with the 3D position of the specific data.

12. The method for scanning according to any of claims 8 to 11, wherein foreign objects in the ear are filtered out from the images of the ear.

13. The method for scanning according to any of claims 8 to 12, wherein foreign objects are presented to the operator on a display, such as wherein the foreign objects are coloured in a different colour on the display than the rest of the surface on the image.

14. A scanner system for three-dimensional scanning of interior surfaces, said scanner system comprising:
- a probe shape scanner (101, 102, 1201) configured to be entered into a cavity, said probe shaped scanner comprising
• at least one light source (202) adapted to create and project structured light producing a pattern on the interior surface of an object, where said light source emits light from a point of emission; and
• at least one camera (205, 505), adapted to record 2D images of the pattern, where the camera accumulates light at a point of accumulation;
- a data conversion device adapted to convert 2D images into 3D real world coordinates,
- a data processing device configured to register one or more holes in the 3D real world coordinates corresponding to areas of the interior surface where visual access is blocked due to the geometrical form of the interior surface, and to provide data and process said data to create 3D real world coordinates for the areas of the interior surface where said visual access is blocked.

## Patentansprüche

1. Verfahren zum Scannen teilweise blockierter Innenflächen, wobei das Verfahren umfasst:
- Bereitstellen eines sondenförmigen Scanners (101, 102, 1201), der eine Achse aufweist, wobei der sondenförmige Scanner umfasst:
• mindestens eine Lichtquelle (202), die dazu eingerichtet ist, strukturiertes Licht zu erzeugen und zu projektieren, und
• mindestens eine Kamera (205, 505), die dazu eingerichtet ist, zweidimensionale Bilder aufzuzeichnen;
- Einbringen des sondenförmigen Scanners in einen Hohlraum eines Objekts (502), wobei der Hohlraum durch eine Innenfläche (502) des Objekts begrenzt wird;
- Erzeugen und Projektieren strukturierten Lichts (501) von der Lichtquelle der Sonde, das ein Muster auf der Innenfläche des Objekts produziert;
- Aufzeichnen einer Serie zweidimensionaler Bilder der Reflektion des Musters von der Innenfläche unter Verwendung der Kamera;
- Kombinieren der Serie zweidimensionaler Bilder, um dreidimensionale Realweltkoordinaten der Innenfläche zu erhalten;
- in Register Bringen eines oder mehrerer Löcher in den dreidimensionalen Realweltkoordinaten, die Gebieten der Innenfläche entsprechen, wo aufgrund der geometrischen Form der Innenfläche der visuelle Zugang blockiert ist; und
- Bereitstellen von Daten und Verarbeiten dieser Daten, um dreidimensionale Realweltkoordinaten für die Gebiete der Innenfläche zu erzeugen, wo der besagte visuelle Zugang blockiert ist.

2. Verfahren zum Scannen nach Anspruch 1, wobei ein Teil der Daten von Oberflächeninformation des linken Ohrs als Teil der Daten von Oberflächeninformation für das rechte Ohr verwendet wird, wenn Teile des rechten Ohrs vorliegen, wo keine Oberflächeninformation ermittelt wurde, und umgekehrt.

3. Verfahren zum Scannen nach einem der vorhergehenden Ansprüche, wobei Löcher in einem Scan auf Basis vorhergehender Scans des Ohrs der Person abgeleitet oder interpoliert werden.

4. Verfahren zum Scannen nach dem vorhergehenden Anspruch, wobei das Verfahren umfasst:
- Vorsehen, dass die Kamera (205, 505) eine Vielzahl von Sensorelementen oder eine Vielzahl von Gruppen von Sensorelementen umfasst;
- Variieren der Fokusebene des Musters über einen Bereich von Fokusebenenpositionen unter Aufrechterhaltung eines im Wesentlichen feststehenden räumlichen Verhältnisses der Kamera und der Innenfläche,
wobei das Kombinieren zweidimensionaler Bilder das Ermitteln, durch Analyse der zweidimensionalen Bilder, der In-Fokus-Positionen der Fokusebene umfasst für:
a) jedes einer Vielzahl von Sensorelementen in der Kamera für den Bereich von Fokusebenenpositionen, oder
b) jede einer Vielzahl von Gruppen der Sensorelemente in der Kamera für den Bereich von Fokusebenen,
und wobei die dreidimensionalen Realweltkoordinaten von den In-Fokus-Positionen stammen.

5. Verfahren zum Scannen nach einem der vorhergehenden Ansprüche, wobei Löcher in der Oberflächeninformation geschlossen werden, indem sie an mathematische Oberflächen höherer Ordnung, wie etwa zweiter Ordnung, dritter Ordnung, vierter Ordnung etc., angepasst werden.

6. Verfahren zum Scannen nach einem der vorhergehenden Ansprüche, wobei Löcher in der Oberflächeninformation geschlossen werden, indem Information verwendet wird darüber, wo keine Oberfläche vorhanden ist.

7. Verfahren zum Scannen nach einem der vorhergehenden Ansprüche, wobei Löcher in der Oberflächeninformation geschlossen werden, indem andere Daten als Bilddaten verwendet werden, wie etwa Farbe, Interferenz, Winkel des reflektierten Lichts, und/oder Daten von einem oder mehr anderen Sensoren als der Kamera.

8. Verfahren zum Scannen nach einem der vorhergehenden Ansprüche, wobei Fremdkörper im Gehörgang identifiziert werden.

9. Verfahren zum Scannen nach Anspruch 8, wobei das Identifizieren von Fremdkörpern das Detektieren von Reflektionen von dem Fremdkörper und Analysieren der Differenz in dem reflektierten Licht von der Haut beziehungsweise dem Fremdkörper umfasst.

10. Verfahren zum Scannen nach einem der vorhergehenden Ansprüche, wobei das Verfahren die Anwendung von monochromem Licht zur Beleuchtung der Oberfläche zum Analysieren von reflektiertem Licht umfasst.

11. Verfahren zum Scannen nach einem der Ansprüche 8 bis 10, wobei das Verfahren das Identifizieren von Fremdkörpern durch Korrelieren von Oberflächendaten mit der dreidimensionalen Position der spezifischen Daten umfasst.

12. Verfahren zum Scannen nach einem der Ansprüche 8 bis 11, wobei Fremdkörper im Ohr aus den Bildern des Ohrs ausgefiltert werden.

13. Verfahren zum Scannen nach einem der Ansprüche 8 bis 12, wobei Fremdkörper der Bedienperson auf einer Anzeige dargestellt werden, wie etwa wobei die Fremdkörper auf der Anzeige in einer anderen Farbe gefärbt sind als der Rest der Oberfläche auf dem Bild.

14. Scannersystem zum dreidimensionalen Scannen von Innenflächen, wobei das Scannersystem umfasst:
- einen sondenförmigen Scanner (101, 102, 1201), der dazu eingerichtet ist, in einen Hohlraum eingebracht zu werden, wobei der sondenförmige Scanner umfasst:
• mindestens eine Lichtquelle (202), die dafür ausgelegt ist, strukturiertes Licht zu erzeugen und zu projektieren, das ein Muster auf der Innenfläche eines Objekts produziert, wobei die Lichtquelle Licht von einem Emissionspunkt aus abstrahlt; und
• mindestens eine Kamera (205, 505), die dafür ausgelegt ist, zweidimensionale Bilder des Musters aufzuzeichnen, wobei die Kamera Licht an einem Sammelpunkt sammelt;
- eine Datenumsetzungseinrichtung, die dafür ausgelegt ist, zweidimensionale Bilder in dreidimensionale Realweltkoordinaten umzusetzen,
- eine Datenverarbeitungseinrichtung, die dazu eingerichtet ist, ein oder mehr Löcher in den dreidimensionalen Realweltkoordinaten zu Gebieten der Innenfläche, wo aufgrund der geometrischen Form der Innenfläche der visuelle Zugang blockiert ist, in Register zu bringen, und Daten bereitzustellen und diese Daten zu verarbeiten, um dreidimensionale Realweltkoordinaten für die Gebiete der Innenfläche, wo der besagte visuelle Zugang blockiert ist, zu erzeugen.

## Revendications

1. Procédé de balayage de surfaces intérieures partiellement obstruées, le procédé comprenant :
- fournir un scanner en forme de sonde (101, 102, 1201) ayant un axe, le scanner en forme de sonde comprenant :
• au moins une source lumineuse (202) configurée pour créer et projeter une lumière structurée, et ;
• au moins une caméra (205, 505) configurée pour enregistrer des images 2D ;
- faire entrer ledit scanner en forme de sonde dans une cavité d'un objet (502), ladite cavité étant délimitée par une surface intérieure (502) de l'objet ;
- créer et projeter une lumière structurée (501) à partir de ladite source de lumière de la sonde produisant un motif sur la surface intérieure de l'objet ;
- enregistrer une série d'images 2D de la réflexion du motif à partir de la surface intérieure en utilisant ladite caméra ;
- combiner ladite série d'images 2D pour obtenir des coordonnées 3D de l'environnement réel de la surface intérieure ;
- enregistrer un ou plusieurs trous dans les coordonnées 3D de l'environnement réel correspondant aux zones de la surface intérieure où l'accès visuel est empêché en raison de la forme géométrique de la surface intérieure ; et
- fournir des données et traiter lesdites données pour créer des coordonnées 3D de l'environnement réel pour les zones de la surface intérieure où ledit accès visuel est empêché.

2. Procédé de balayage selon la revendication 1, dans lequel une partie des données d'informations de surface de l'oreille gauche est utilisée comme partie des données d'informations de surface pour l'oreille droite, s'il existe des parties de l'oreille droite où les informations de surface n'ont pas été acquises, et vice versa.

3. Procédé de balayage selon l'une quelconque des revendications précédentes, dans lequel des trous dans un balayage sont déduits ou interpolés sur la base de balayages antérieurs de l'oreille de la personne.

4. Procédé de balayage selon la revendication précédente, dans lequel le procédé comprend :
- prévoir que la caméra (205, 505) comprenne une pluralité d'éléments de détection ou une pluralité de groupes d'éléments de détection ;
- faire varier le plan de focalisation du motif sur une plage de positions de plan de focalisation tout en maintenant une relation spatiale sensiblement fixe de la caméra et de ladite surface intérieure ;
dans lequel la combinaison d'images 2D comprend la détermination par analyse desdites images 2D des positions de focalisation du plan de focalisation pour :
a) chaque élément parmi une pluralité d'éléments de détection dans la caméra pour ladite plage de positions de plan de focalisation, ou
b) chaque groupe parmi une pluralité de groupes d'éléments de détection dans la caméra pour ladite plage de plan de focalisation ;
et lesdites coordonnées 3D de l'environnement réel sont issues desdites positions de focalisation.

5. Procédé de balayage selon l'une quelconque des revendications précédentes, dans lequel les trous dans les informations de surface sont fermés en s'ajustant à des surfaces mathématiques d'ordre supérieur, telles que des ordres de second ordre, de troisième ordre, de quatrième ordre, etc.

6. Procédé de balayage selon l'une quelconque des revendications précédentes, dans lequel les trous dans les informations de surface sont fermés en utilisant des informations sur des endroits où il n'existe aucune surface.

7. Procédé de balayage selon l'une quelconque des revendications précédentes, dans lequel les trous dans les informations de surface sont fermés en utilisant d'autres données que des données d'image, telles que couleur, interférence, angle de lumière réfléchie et/ou données d'un ou plusieurs autres détecteurs que la caméra.

8. Procédé de balayage selon l'une quelconque des revendications précédentes, dans lequel des objets étrangers dans le conduit de l'oreille sont identifiés.

9. Procédé de balayage selon la revendication 8, dans lequel l'identification d'objets étrangers comprend la détection de réflexions de l'objet étranger, et l'analyse de la différence de la lumière réfléchie par la peau et l'objet étranger, respectivement.

10. Procédé de balayage selon l'une quelconque des revendications précédentes, dans lequel le procédé comprend l'utilisation d'une lumière monochromatique pour éclairer la surface pour analyser la lumière réfléchie.

11. Procédé de balayage selon l'une quelconque des revendications 8 à 10, dans lequel le procédé comprend l'identification d'objets étrangers en corrélant des données de surface avec la position 3D des données spécifiques.

12. Procédé de balayage selon l'une quelconque des revendications 8 à 11, dans lequel des objets étrangers dans l'oreille sont filtrés à partir des images de l'oreille.

13. Procédé de balayage selon l'une quelconque des revendications 8 à 12, dans lequel des objets étrangers sont présentés à l'opérateur sur un affichage, notamment avec les objets étrangers colorés dans une couleur différente sur l'affichage que le reste de la surface sur l'image.

14. Système de balayage pour un balayage tridimensionnel de surfaces intérieures, ledit système de balayage comprenant :
- un scanner en forme de sonde (101, 102, 1201) configuré pour entrer dans une cavité, ledit scanner en forme de sonde comprenant :
• au moins une source lumineuse (202) adaptée à créer et projeter une lumière structurée produisant un motif sur la surface intérieure d'un objet, ladite source de lumière émettant de la lumière à partir d'un point d'émission ; et
• au moins une caméra (205, 505), adaptée à enregistrer des images 2D du motif, la caméra accumulant de la lumière à un point d'accumulation ;
- un dispositif de conversion de données adapté à convertir des images 2D en coordonnées 3D de l'environnement réel ;
- un dispositif de traitement de données configuré pour enregistrer un ou plusieurs trous dans les coordonnées 3D de l'environnement réel correspondant à des zones de la surface intérieure où l'accès visuel est empêché à cause de la forme géométrique de la surface intérieure, et pour fournir des données et traiter lesdites données pour créer des coordonnées 3D de l'environnement réel pour les zones de la surface intérieure où ledit accès visuel est empêché.
